# EUROPEAN PATENT APPLICATION

(11) **EP 3 992 208 A1**
(43) Date of publication of application: **04.05.2022**
(21) Application number: 20205559.6
(22) Date of filing: 03.11.2020
(51) Int. Cl.: C07K 16/28

(54) **ANTI-PD-L1 VHH ANTIBODIES**

(71) Applicant: Randox Laboratories Ltd, Crumlin, Antrim BT29 4QY (GB)
(72) Inventor: Dunlop, Marshall, Crumlin, Antrim BT29 4QY (GB); Schon, Shirley, Crumlin, Antrim BT29 4QY (GB); Lamont, John, Crumlin, Antrim BT29 4QY (GB); Fitzgerald, Stephen, Crumlin, Antrim BT29 4QY (GB)
(74) Representative: Gill Jennings & Every LLP

(57) **Abstract**

The present invention relates to therapeutic antibodies, or antigen-binding portions thereof, for use in the treatment of cancer and infectious disease. Specifically, the present invention relates to therapeutic antibodies, or antigen-binding portions thereof, that target the programmed death-ligand 1 polypeptide. The present invention also relates to pharmaceutical compositions comprising said therapeutic antibody, or antigen-binding portions thereof.

## Description

### Field of the Invention

The present invention relates to binding molecules with specificity for immune checkpoint molecules. The present invention also relates to pharmaceutical compositions comprising the binding molecules for use in the treatment of cancer and infectious disease.

### Background

Immune checkpoint molecules are molecules that regulate the activity of the immune system. The ability to self-regulate the activity of immune cell types is crucial in host survival; protecting the host against excessive tissue damage and preventing the development of autoimmune diseases; a group of diseases characterised by the immune system indiscriminately attacking the body's cells as opposed to cells of foreign origin.

Immune checkpoint molecules may be stimulatory or inhibitory, allowing the creation of a highly regulated homeostatic system in which immune cell type activity can be controlled. As a primary mediator of immune effector function, activated T-cells are one type of immune cell that are subjected to such control via the expression of multiple inhibitory immune checkpoint molecules. Examples of such molecules include programmed cell death protein-1 (PD-1), cytotoxic T-lymphocyte-associated protein 4 (CTLA-4) and lymphocyte-activation gene 3 (LAG-3).

Programmed Cell Death Protein 1, (PD-1 or CD279), a 55-kD type 1 transmembrane protein, is a member of the CD28 family of T cell co-stimulatory receptors that include immunoglobulin superfamily member CD28, CTLA-4, inducible co-stimulator (ICOS), and BTLA. PD-1 is highly expressed on activated T cells and B cells. PD-1 expression can also be detected on memory T-cell subsets with variable levels of expression. Two ligands specific for PD-1 have been identified: programmed death- ligand 1 (PD-L1, also known as B7-H1 or CD274) and PD-L2 (also known as B7-DC or CD273). PD-L1 and PD-L2 have been shown to down-regulate T cell activation upon binding to PD-1 in both mouse and human systems (Okazaki et al., Int Immunol., 2007; 19: 813-824). The interaction of PD-1 with its ligands, PD-L1 and PD-L2, which are expressed on antigen-presenting cells (APCs) and dendritic cells (DCs), transmits negative regulatory stimuli to down-modulate the activated T cell immune response. Activation of either of these ligands leads to dephosphorylation and inactivation of the T-cell kinase ZAP70 and the recruitment of SHP2. SHP2 directly dephosphorylates Pl3K, inhibiting the downstream activation of Akt and ultimately leads to a reduction in inflammatory cytokines and cell survival proteins, triggering apoptotic pathways and leading to the destruction of the T-cell. Blockade of PD-1 suppresses this negative signal and amplifies T cell responses.

Despite the purpose of this regulatory system being to protect the host, numerous scientific studies have demonstrated that the tumour microenvironment is capable of hijacking this system. For example, numerous studies indicate that the cancer microenvironment manipulates the PD-L1-/PD-1 signalling pathway and that induction of PD-L1 expression is associated with inhibition of immune responses against cancer, thus permitting cancer progression and metastasis. The PD-L1/PD-1 signalling pathway is a primary mechanism of cancer immune evasion for several reasons. First, and most importantly, this pathway is involved in negative regulation of immune responses of activated T effector cells, found in the periphery. Second, PD-L1 is up-regulated in cancer microenvironments, while PD-1 is also up-regulated on activated tumour infiltrating T cells, thus possibly potentiating a vicious cycle of inhibition. Third, this pathway is intricately involved in both innate and adaptive immune regulation through bi-directional signalling. These factors make the PD-1/PD-L1 complex a central point through which cancer can manipulate immune responses and promote its own progression. As a result, the tumour is able to activate inhibitory immune checkpoint molecule pathways, resulting in a suppressed immune system and the continued unimpeded growth of cancerous cells. Similarly, growing evidence in the scientific community points toward the exploitation of immune checkpoints in infectious disease, for example, HIV, tuberculosis and malaria. Consequently, the prevention of this interaction between immune cells and tumour cells has been a focus of therapeutic intervention in the cancer field for quite some time and now represents a growing area of interest in the infectious disease field.

A number of monoclonal antibodies have been developed to target either PD-1 or PD-L1; these include Pembrolizumab (Keytruda), Nivolumab (Opdivo), Cemiplimab (Libtayo), Avelumab (Bavencio) Atezolizumba (Tecentriq) and Durvalumab (Imfinzi). These drugs have been shown to be beneficial in treating a number of different cancers, including melanoma of the skin, non-small cell lung cancer and Hodgkin lymphoma.

A noted side-effect of the existing immune checkpoint inhibitor therapies is the occurrence of severe immune-related adverse events (IRAEs). One strategy to minimise the risk of these types of side-effects is to limit the exposure of the immunotherapy to systemic circulation. This can be achieved through localised administration of the immunotherapy to the tumour microenvironment (TME). However, even with this targeted administration, the immunotherapeutic agent inevitably leaches out of the TME into the systemic circulation, where they can remain for weeks, resulting in a systemic reduction in immune self-tolerance.

Accordingly, there is a need in the immunotherapy field for an efficacious immunotherapy directed at immune checkpoint molecules that are able to retain their efficacy whilst limiting any immune-related adverse events and limiting the potential of a systemic reduction in immune self-tolerance.

### Summary of the invention

The present invention relates to therapeutic antibodies, or antigen-binding portions thereof, that target the programmed death-ligand 1 polypeptide in the context of cancer and infectious disease. The inventors of the present invention have surprisingly found that the antibodies, or antigen-binding portions thereof, herein disclosed display an impressive level of efficacy whilst limiting any immune-related adverse events. The applicant is unaware of any other immune checkpoint inhibitor that currently possesses this combination of properties.

In a first aspect, the present invention provides for an antibody or antigen-binding portion thereof, wherein the antibody or antigen-binding portion thereof has at least 80 % sequence identity to SEQ ID NO: 7 and wherein the antibody or antigen-binding portion thereof can specifically bind a programmed death-ligand 1 (PD-L1) polypeptide.

In a second aspect, the present invention provides for an antibody or antigen-binding portion thereof, wherein the antibody or antigen-binding portion thereof comprises one or more heavy chain complementarity-determining regions (CDRs), wherein (i) the heavy chain CDR1 has at least 70% sequence identity to SED ID NO: 1, (ii) the heavy chain CDR2 has at least 70% sequence identity to SEQ ID NO: 2 and (iii) the heavy chain CDR3 has at least 70% sequence identity to SEQ ID NO:3, wherein the antibody or antigen-binding portion thereof can specifically bind a programmed death-ligand 1 (PD-L1) polypeptide.

In a third aspect, the present invention provides for an antibody or antigen-binding portion thereof, wherein the antibody or antigen-binding portion thereof comprises a heavy chain CDR having at least 70 % sequence identity to SEQ ID NO: 3.

In a fourth aspect, the present invention provides for a nucleic acid encoding the antibody, or antigen-binding portion thereof, as defined in the first aspect of the present invention, or a complementary sequence thereof.

In a fifth aspect, the present invention provides for a pharmaceutical composition comprising the antibody or nucleic acid disclosed herein and medical uses thereof.

### Brief Description of the Figures

The invention is defined with reference to the accompanying drawings, wherein:
**Figure 1** shows a schematic demonstrating a PD-1/PD-L1 inhibition ELISA.
**Figure 2** shows the results obtained via an ELISA-based assay, demonstrating the ability of selected sdAb clones to block the interaction between PD-1 and PD-L1.
**Figure 3** shows the results obtained via an inhibition assay, demonstrating the ability of selected sdAb clones to block PD1/PD-L1 interaction.
**Figure 4** shows the results obtained from a PD-L1 binding assay for the humanised 32.1A1 clone.
**Figure 5** shows the results obtained from a PD-1/PD-L1 inhibition assay to compare clone 32.1A1, its humanised derivative and its close relative 32.2F7 to the commercially available therapeutic antibody Avelumab.
**Figure 6** shows a schematic demonstrating the PD-1/PD-L1 blockade bioassay.
**Figure 7** shows the luciferase activity fold change of the two lead PD-1 and PD-L1 blocking clones, as identified by the ELISA inhibition assay, as assayed in the Promega PD-1/PD-L1 blockade bioassay. Reference to GS542 denotes the humanised 32.1A1 clone, and reference to 1A1 denotes clone 32.1A1.
**Figure 8** shows an SDS-PAGE gel showing fraction of sdAb monomer.
**Figure 9** shows an analytical size exclusion chromatography (SEC) to demonstrate aggregation following thermal stress of the humanised 32.1A1 clone.
**Figure 10** shows humanised 32.1A1 stability ELISA against coated PD-L1.
**Figure 11** shows cross-reactivity of clone 32.1A1 against related and unrelated antigens.

### Detailed Description

The present invention relates to therapeutic antibodies, or antigen-binding portions thereof, that target the programmed death-ligand 1 polypeptide in the context of cancer and infectious disease.

In order that the present invention may be more readily understood, certain terms are first defined. Additional definitions are set forth throughout the detailed description.

A "checkpoint inhibitor" is an agent, which acts on surface proteins, which are members of either the TNF receptor or B7 superfamilies, including agents which bind to negative co-stimulatory molecules selected from CTLA-4, PD-1, TIM-3, BTLA, VISTA, LAG-3, and/or their respective ligands, including PD-L1.

The term "specifically binds", "specific binding" or "binds" are used interchangeably and refers to an antibody, or antigen-binding portion thereof, binding to an antigen or an epitope of said antigen with greater affinity than for other antigens or epitopes. Such affinity is measured via the equilibrium dissociation constant (Kd) and may be measured using standard procedures known in the art. Antibodies or antigen-binding portions herein disclosed may have cross-reactivity to other related antigens, for example, to the same antigen from other species (homologs).

The terms "PD-1", "PD1", "programmed death 1", "programmed cell death 1" are used interchangeably and taken to include variants, isoforms and species homologs of human PD-1. The complete PD-1 sequence can be found under GenBank Accession No. U64863.

The terms "PD-L1", "PDL1", "programmed death ligand 1" and "programmed cell death 1" are used interchangeably and taken to include variants, isoforms and species homologs of human PD-L1. The complete PD-L1 sequence can be found under GenBank Accession No. NP 054862.1.

The term "antibody" as used herein refers to an immunoglobulin or immunoglobulin-like molecule that binds to an epitope on a target protein, for example, PD-L1.

The term "monoclonal antibody" as used herein refer to a preparation of antibody molecules of single molecular composition. A monoclonal antibody composition displays a single binding specificity and affinity for a particular epitope.

The term "human antibody", as used herein, is intended to include antibodies having variable regions in which both the framework and CDR regions are derived from human germline immunoglobulin sequences. The term "humanized antibody" is intended to refer to antibodies in which CDR sequences derived from the germline of another mammalian species, such as a mouse, have been grafted onto human framework sequences. Additional framework region modifications may be made within the human framework sequences.

The term "complementarity-determining regions" and "CDRs" are used interchangeably and refer to the antigen-binding region (or paratope) of an antibody.

The term 'binding affinity' refers to the strength of the binding interaction between the antibody or antigen-binding portion herein disclosed and the ligand to which it is intended to bind, for example, PD-L1. Binding affinity is typically measured and reported by the equilibrium dissociation constant (Kd). A skilled person in the art will recognise that the smaller the Kd value the greater the binding affinity and therefore a smaller Kd value would be desirable for a binding protein intended as a therapeutic.

As used herein, the terms "fragment", "derivative" and "analog" refer to a polypeptide that substantially retains the same biological function or activity of an antibody of the invention. A polypeptide fragment, derivative or analog of the present invention may be (i) a polypeptide having one or more conservative or non-conservative amino acid residues (preferably conservative amino acid residues) substituted, and such substituted amino acid residues may or may not be encoded by the genetic code, or (ii) a polypeptide having a substituent group in one or more amino acid residues, or (iii) a mature polypeptide with another compound (such as a compound that extends the half-life of a polypeptide, such as (Polyethylene glycol) fusion polypeptide, or (iv) a polypeptide formed by fusing additional amino acid sequences to this polypeptide sequence (such as a leader sequence or a secretion sequence or a sequence or protein sequence used to purify this polypeptide, or 6His tag fusion protein).

In one aspect, the present invention provides for an antibody or antigen-binding portion thereof, wherein the antibody or antigen-binding portion thereof has at least 80 % sequence identity to SEQ ID NO: 7 and wherein the antibody or antigen-binding portion thereof can specifically bind a programmed death-ligand 1 (PD-L1) polypeptide.

Accordingly, the present invention provides for an antibody, or antigen-binding portion thereof, that has at least 80 % sequence identity to SEQ ID NO: 7. Preferably, the antibody, or antigen-binding portion thereof, has at least 85 % sequence identity to SEQ ID NO: 7. Even more preferably, the antibody, or antigen-binding portion thereof, has at least 90 %, at least 91 %, at least 92 %, at least 93 %, at least 94 %, at least 95 %, at least 96 %, at least 97 %, at least 98 %, at least 99 % sequence identity to SEQ ID NO: 7.

In one embodiment, the antibody, or antigen-binding portion thereof, may comprise a heavy chain complementarity-determining region (CDR) having at least 70 % sequence identity to SEQ ID NO: 3. Preferably, the antibody, or antigen-binding portion thereof, has a heavy chain CDR having at least 80 % sequence identity to SEQ ID NO: 3. Even more preferably, the antibody, or antigen-binding portion thereof, has at least 90 %, at least 91 %, at least 92 %, at least 93 %, at least 94 %, at least 95 %, at least 96 %, at least 97 %, at least 98 %, at least 99 % sequence identity to SEQ ID NO: 3.

The antibody, or antigen-binding portion thereof, may further comprise a heavy chain CDR having at least 70 % sequence identity to SEQ ID NO: 1 and/or comprise a heavy chain CDR having at least 70 % sequence identity to SEQ ID NO: 2. Preferably, the antibody, or antigen-binding portion thereof, has a heavy chain CDR having at least 80 % sequence identity to SEQ ID NO: 1 and/or SEQ ID NO: 2. Even more preferably, the antibody, or antigen-binding portion thereof, has a heavy chain CDR having at least 90 %, at least 91 %, at least 92 %, at least 93 %, at least 94 %, at least 95 %, at least 96 %, at least 97 %, at least 98 %, at least 99 % sequence identity to SEQ ID NO: 1 and/or SEQ ID NO: 2.

Accordingly, SEQ ID NO: 7 may comprise the heavy chains CDR1 and CDR3, the heavy chains CDR2 and CDR3, or the heavy chains CDR1 and CDR2. It is preferable that the heavy chain CDR3 according to SEQ ID NO: 3 is included within SEQ ID NO: 7.

In another aspect of the present invention, an antibody or antigen-binding portion thereof is provided, wherein the antibody, or antigen-binding portion thereof, comprises one or more heavy chain CDRs, wherein (i) the heavy chain CDR1 has at least 70% sequence identity to SED ID NO: 1, (ii) the heavy chain CDR2 has at least 70% sequence identity to SEQ ID NO: 2 and (iii) the heavy chain CDR3 has at least 70% sequence identity to SEQ ID NO:3, wherein the antibody or antigen-binding portion thereof can specifically bind a programmed death-ligand 1 (PD-L1) polypeptide.

The present invention provides for an antibody, or antigen-binding portion thereof, that has at least 70 % sequence identity to SEQ ID NO: 1, SEQ ID NO: 2 and SEQ ID NO: 3. Preferably, the antibody, or antigen-binding portion thereof, has at least 80 % sequence identity to SEQ ID NO: 1, SEQ ID NO: 2 and SEQ ID NO: 3. Even more preferably, the antibody, or antigen-binding portion thereof, has at least 90 %, at least 91 %, at least 92 %, at least 93 %, at least 94 %, at least 95 %, at least 96 %, at least 97 %, at least 98 %, at least 99 % sequence identity to SEQ ID NO: 1, SEQ ID NO: 2 and SEQ ID NO: 3.

In yet a further aspect of the present invention, an antibody or antigen-binding portion thereof, wherein the antibody or antigen-binding portion thereof comprises a heavy chain CDR having at least 70 % sequence identity to SEQ ID NO: 3 is provided. Preferably, the antibody, or antigen-binding portion thereof, comprises a heavy chain CDR having at least 70 % sequence identity to SEQ ID NO: 3. Even more preferably, the antibody, or antigen-binding portion thereof, has at least 80 %, at least 90 %, at least 91 %, at least 92 %, at least 93 %, at least 94 %, at least 95 %, at least 96 %, at least 97 %, at least 98 %, at least 99 % sequence identity to SEQ ID NO: 3. The antibody, or antigen-binding portion thereof, may further comprise the heavy chain CDRs having at least 60 % sequence identity to SEQ ID NO:1 and SEQ ID NO: 2.

In one embodiment, the antibody or antigen-binding portion thereof that has at least 70 % sequence identity to SEQ ID NO: 1, SEQ ID NO: 2 and SEQ ID NO: 3 and the antibody, or antigen-binding portion thereof, that has at least 70 % sequence identity to SEQ ID NO: 3, has at least 80 % sequence identity to SEQ ID NO: 7. SEQ ID NO: 7 may comprise the CDR regions according to SEQ ID NOs: 1-3. Alternatively, SEQ ID NO: 7 may comprise the CDR regions according to SEQ ID NOs: 4-6, resulting in the antibody, or antigen-binding portion thereof, according to SEQ ID NO: 8. It is preferred that the heavy chain CDR3 according to SEQ ID NO: 6 is included within SEQ ID NO: 8.

Preferably, the aforementioned antibodies, or antigen-binding portion thereof, have at least 85 % sequence identity to SEQ ID NO: 7. More preferably, the aforementioned antibodies, or antigen-binding portion thereof, have at least 90 %, at least 91 %, at least 92 %, at least 93 % or at least 94 % sequence identity to SEQ ID NO: 7. Even more preferably, the aforementioned antibodies, or antigen-binding portion thereof, have at least 95 %, at least 96 %, at least 97 %, at least 98 % or at least 99% sequence identity to SEQ ID NO: 7.

In preferred embodiments, the % sequence identity of SEQ ID NO: 7 retains the sequences of the CDR regions according to SEQ ID NOs: 1-3 (or SEQ ID NOs: 4-6 in relation to SEQ ID NO: 8). In other embodiments, the sequences of the CDR regions may have a combined total of up to 10, preferably up to 8, more preferably up to 5 and most preferably up to 3 amino acid differences compared to the amino acid sequence of SEQ ID NO: 7. Said differences may be present in 1 or more CDR regions. As long as the antibody according to SEQ ID NO: 7 retains its function, the positioning of said differences are not important.

To calculate % homology of any of the sequences herein disclosed, sequence comparison software may be used, for example, using the default settings on the BLAST software package.

In all aspects of the invention, variants of each of the SEQ ID NOs are disclosed. These variants include, but are not limited to, deletions, insertions and/or substitutions of one or more amino acids, and addition of one or several amino acids at the C-terminus and/or N-terminus.

The variant forms of the polypeptides disclosed include, but are not limited to, homologous sequences, conservative variants (wherein there is one or more conservative substitutions), allelic variants, natural mutants, induced mutants, and DNA sites capable of hybridizing with the encoding DNA of the antibody of the invention under high or low stringency conditions, encoded protein, and a polypeptide or protein obtained using an antiserum of an antibody of the invention.

Therefore, the present invention includes variants of the polypeptides according to SEQ ID NOs 1-8. For example, variants of the full-length antibody are disclosed as well as antibodies comprising the CDR regions described above. The variants are considered part of the present disclosure if they display the same function as the antibodies of the invention. These variants include, but are not limited to, deletions, insertions and/or substitutions of one or more amino acids, and addition of one or several amino acids at the C-terminus and/or N-terminus. For example, substitution of amino acids with similar properties is unlikely to change the function of the protein. As another example, adding one or more amino acids to the C-terminus and/or N-terminus is unlikely to change the function of the protein. For example, the present invention also discloses an antibody, or antigen-binding portion thereof comprising the CDRs according to SEQ ID NO: 4, SEQ ID NO: 5 and SEQ ID NO: 6, wherein the substitutions of amino acids from SEQ ID NO: 2 and 3 are highlighted in bold. In one embodiment of the present invention, the antibodies, or antigen-binding portions thereof herein disclosed, may have a Kd value of between 0.3 and 0.6 nM, preferably between 0.4 and 0.6 nM, even more preferably between 0.4 and 0.5 nM. The inventors have surprisingly found that the present invention has a superior Kd value to the commercial anti-PDL1 monoclonal antibody, Avelumab, which has a Kd value of 0.7 nM. Accordingly, the present invention provides for a binding molecule that has an excellent efficacy and toxicity profile in relation to the development of severe immune-related adverse events (IRAEs).

In another embodiment, the antibodies, or antigen-binding portions thereof, may be a single domain antibody. Preferably, the antibodies, or antigen-binding portions thereof, may be a single heavy-chain variable domain comprising the CDRs herein disclosed. These variable regions of the heavy chains of the antibodies of the invention are of particular interest because at least part of them are involved in binding antigens. However, it is understood that any type of immunoglobulin molecule, or fragment thereof, can be used. Examples of other types of immunoglobulin molecules that may be used include, but are not limited to, monoclonal antibodies, Fab fragments, scFv fragments and any other antigen binding fragments provided that incorporate the CDRs herein disclosed.

The present invention also provides for other polypeptides, wherein the antibodies, or antigen-binding portions thereof, form part of a fusion protein or a bispecific antibody. By "fusion protein" we refer to any protein consisting of at least two domains that are encoded by separate genes that have been joined so that they are transcribed and translated as a single unit, thereby producing a single polypeptide. Accordingly, the present invention discloses a fusion protein having the properties of the antibody, or antigen-binding portion thereof, herein disclosed as well as additional properties derived from the additional original protein. Examples of fusion proteins include the fusion of fluorescent tags or Fc tags to the antibody, or antigen-binding portion thereof, of the present invention. By "bispecific antibody" we refer to an artificial protein that combines the specificities of two antibodies, or antibody-like binding protein, into one molecule that can bind to two different antigens or epitopes. Therefore, the present invention also discloses a bispecific antibody wherein one of the binding entities of the bispecific antibody is the antibody, or antigen-binding portion thereof, herein disclosed. It is therefore envisaged that the bispecific antibody, in addition to targeting PD-L1, may also target additional immune checkpoint inhibitors, for example, CTLA-4, PD-1, TIM-3, BTLA, VIST or LAG-3 or any other immune-related or non-immune related molecules involved in disease pathology. The use of bispecific antibodies may be particularly useful given the multifactorial nature of many diseases, in particular cancer.

The invention also includes fragments, derivatives, and analogues of the antibodies, or antigen-binding portions thereof, herein disclosed. Therefore, in addition to almost full-length polypeptides, in some embodiments the invention also includes fragments of the antibodies of the invention. Generally, the fragment has at least about 50 consecutive amino acids, preferably at least about 50 consecutive amino acids, more preferably at least about 80 consecutive amino acids, and most preferably at least about 100 consecutive amino acids in an antibody of the invention.

In a preferred embodiment, the antibodies or antigen-binding portions thereof may be a human or humanised antibody, or antigen-binding portion thereof. Preferably, the antibodies, or antigen-binding portions thereof, is a human antibody or antigen-binding portion thereof. It is understood that the type of antibody employed will be dependent on the intended end user, i.e. human or non-human.

In a further aspect, the present invention provides for nucleic acids/nucleotide sequences encoding the antibodies or antigen-binding portions thereof, or a complementary sequence thereof. The nucleic acids/nucleotide sequences may, for example, be DNA or RNA and may or may not contain intronic sequences. Yet further, the present invention provides a nucleic acid that hybridises to the nucleic acid of the present invention under low and/or high stringency conditions. By 'high stringency conditions' we include high temperature and low salt content in hybridisation buffers. By 'low stringency conditions' we include lower temperatures and high salt content in hybridisation buffers.

In an embodiment, the antibody or antigen-binding portion or nucleic acid thereof may be for use in therapy. It is envisaged that said antibody or antigen-binding portion thereof may be used as therapy in the treatment of any disease or condition in a subject that would arise from pathological PD-L1 signalling and wherein, as a result, the subject would benefit from the blockade of said signalling. The term "therapy" or "treatment" refers to administering an active agent with the purpose to cure, heal, alleviate, relieve, alter, remedy, ameliorate, improve or affect a condition (e.g. a disease), the symptoms of the condition, or to preventor delay the onset of symptoms, complications, biochemical indicia of a disease, or otherwise arrest or inhibit further development of the disease, condition, or disorder in a statistically significant way.

In a further embodiment, the antibodies, or antigen-binding portions or nucleic acid thereof, may be for use in the treatment of cancer. The terms "cancer" and "tumour" are used interchangeably and refer to a cell or population of cells whose growth, proliferation or survival is greater than growth, proliferation or survival of a normal counterpart cell, e.g. a cell proliferative or differentiative disorder. Typically, the growth is uncontrolled. The term 'malignancy' refers to the invasion of nearby tissue by the cancerous cells.

In a preferred embodiment, the cancer may be a tumour present within the central nervous system (CNS). It is envisaged that the antibody or antigen-binding portion thereof herein disclosed may be of particular use in cancers of the CNS owing to its smaller size. As a result of the smaller size of the antibody or antigen-binding portion thereof, there is a greater potential for it to be engineered to cross the blood-brain barrier compared to antibody molecules of a larger size, for example, IgGs.

In yet another preferred embodiment, the cancer that the present invention is intended to treat is a solid tumour. By "solid tumour" we intend an abnormal mass of tissue that typically lacks cysts or areas of liquid. It is intended that cancers that are particularly responsive to immunotherapy intervention may be targeted. Accordingly, subject samples may be collected to determine expression levels of, for example, PD-L1 on tumour cells to determine if an antibody directed to PD-L1 would be efficacious.

Examples of cancers which may be treated according to the present invention include, but are not limited to: melanoma, renal cancer, prostate cancer, breast cancer, colon cancer, lung cancer, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the oesophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukaemia's including acute myeloid leukaemia, chronic myeloid leukemia, acute lymphoblastic leukemia, chronic lymphocytic leukemia, solid tumours of childhood, lymphocytic lymphoma, cancer of the bladder, cancer of the kidney or ureter, carcinoma of the renal pelvis, neoplasm of the central nervous system (CNS), primary CNS lymphoma, tumour angiogenesis, spinal axis tumour, brain stem glioma, pituitary adenoma, Kaposi's sarcoma, epidermoid cancer, squamous cell cancer, T-cell lymphoma, and combinations of said cancers. The present invention is also suitable for use in metastatic cancers, particularly those which are known to express PD-L1.

In yet a further embodiment, the present invention may be for use in the treatment of infectious disease. The term 'infectious disease' refers to a group of disorders caused by microorganisms; bacteria, viruses, fungi or parasites. The diseases caused by each may be spread directly or indirectly. The infectious disease may be a viral infection, a bacterial infection, a parasitic infection or a fungal infection.

Examples of viral infections that may be treated include, but are not limited to, human immunodeficiency virus (HIV), ebola virus, hepatitis virus (A, B, or C), herpes virus (e.g, VZV, HSV-I, HAV-6, HSV-II, and CMV, Epstein Barr virus), adenovirus, influenza virus, flaviviruses, echovirus, rhinovirus, coxsackie virus, coronavirus, respiratory syncytial virus, mumps virus, rotavirus, measles virus, rubella virus, parvovirus, vaccinia virus, HTLV virus, dengue virus, papillomavirus, molluscum virus, poliovirus, rabiesvirus, JC virus or arboviral encephalitis virus.

Examples of possible bacterial infections include, but are not limited to, *Chlamydia,* rickettsial bacteria, mycobacteria, staphylococci, streptococci, pneumonococci, meningococci and gonococci, klebsiella, proteus, serratia, pseudomonas, *Legionella, Corynebacterium diphtheriae, Salmonella,* bacilli, *Vibrio cholerae, Clostridium tetan, Clostridium botulinum, Bacillus anthricis, Yersiniapestis, Mycobacterium leprae, Mycobacterium lepromatosis, and Borriella.* Examples of possible parasitic infections to be treated include *Entamoeba histolytica, Balantidium coli, Naegleria fowleri, Acanthamoeba, Giardia lambia, Cryptosporidium, Pneumocystis carinii, Plasmodium vivax, Babesia microti, Trypanosoma brucei, Trypanosoma cruzi, Leishmania donovani, Toxoplasma gondii, Nippostrongylus brasiliensis.* Examples of possible fungal infections to be treated include *Candida (albicans, krusei, glabrata, tropicalis, etc.), Cryptococcus neoformans, Aspergillus (fumigatus, niger, etc.),* Genus *Mucorales (mucor, absidia, rhizopus), Sporothrix schenkii, Blastomyces dermatitidis, Paracoccidioides brasiliensis, Coccidioides immitis and Histoplasma capsulatum.*

The present invention provides for a pharmaceutical composition comprising the antibody, or antigen-binding portion thereof or the nucleic acid herein disclosed. In some embodiments, the pharmaceutical composition comprises a therapeutically effective amount of the present invention, in combination with a pharmaceutically acceptable carrier and/or a known therapeutic agent. In other embodiments, the pharmaceutical composition comprises a sub-therapeutic amount of the present invention, in combination with a pharmaceutically acceptable carrier and/or a known therapeutic agent. In the latter case, it is envisaged that the antibody or antigen-binding portion thereof herein disclosed may act in a synergistic manner with additional therapeutic agents. This may require lower doses of each therapeutic agent and is thus expected to reduce the incidence of any adverse effects that the subject may experience following said therapy/treatment.

The term "therapeutically effective amount" refers to the amount of the antibody that preferably results in a decrease in severity of disease symptoms, an increase in frequency and duration of disease symptom-free period, or a prevention of impairment or disability due to the disease affliction (i.e. cancer or infectious disease).

The term "effective amount" refers to the sufficient amount of an agent required to provide the desired biological or therapeutic result. That result can be reduction, amelioration, palliation, lessening, delaying, and/or alleviation of one or more of the signs, symptoms or causes of a disease or any other desired alteration of a biological system.

In reference to cancer, an effective amount may comprise an amount sufficient to cause a tumour to shrink and/or to decrease the growth rate of the tumour (such as to suppress tumour growth) or to prevent or delay other unwanted cell proliferation. In some embodiments, an effective amount sufficient to delay development, or prolong survival or induce stabilisation of the cancer or tumour.

In reference to infectious disease, an effective amount may comprise a reduction in viral load and or an increase in the subjects CD4 count (a measure indicative of the strength of the immune system).

A pharmaceutically acceptable carrier is intended to refer to any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration. Depending on the route of administration, the administered product may be coated in a material to protect the product from adverse physiological conditions that may result in inactivation of the product (for example, high acidity).

The pharmaceutical composition may comprise a pharmaceutically acceptable salt; this term refers to a salt that retains the desired biological activity of the parent compound and does not impart any undesired toxicological effects.

Examples of such salts include acid addition salts and base addition salts. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N'-dibenzylethylenediamine, N-methylglucamine,chloroprocaine, choline, diethanolamine, ethylenediamine, procaine and the like.

The pharmaceutical composition of the present invention may also include a pharmaceutically acceptable anti-oxidant. Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Suitable aqueous and non-aqueous carriers that may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

The pharmaceutical compositions may further contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents that delay absorption such as aluminium monostearate and gelatin.

The present invention includes a pharmaceutical composition that may optionally include an additional therapeutic agent, known to be efficacious in, for example, cancer or infectious disease. For example, the present invention may be used in combination with known chemotherapeutic agents, for example, altretamine, busulfan, carboplatin, carmustin, cisplatin and decarbazine, as a method to reduce their levels of toxicity. The present invention may also be used in combination with infectious disease therapeutics, for example, antiretroviral therapy, antibiotics, antifungals and antiparasitics. The pharmaceutical composition may be given concurrently/simultaneously to additional therapies, for example, radiotherapy or chemotherapy for subjects with cancer. The term "concurrently" or "simultaneously" refers to administration of said therapies occurring on the same day. Alternatively, the pharmaceutical composition may be given separately to additional therapies. The term "separately" refers to administration of said therapies occurring on different days.

The present invention may also include the presence of one or more immunogenic agents. By "immunogenic agents" we intend any molecule that is capable of inducing an immune response. Examples of such immunogenic agents include cancerous cells, purified tumour antigen and cells transfected with genes encoding immune stimulating cytokines. The use of these agents in combination with the antibody disclosed herein may be used to generate an enhanced immune response against a target, for example, tumour cells.

The invention will now be illustrated in the following examples with reference to the accompanying drawings.

### Example 1 - Generation of binding clones

Six male alpacas were immunised with recombinant human PD1 (Randox) whilst a further six alpacas were immunised with recombinant human PD-L1 (Randox). All 12 animals were immunised at monthly intervals for 7 months. Lymph node cells were harvested, and single domain antibody libraries were generated by RT-PCR using published primer sets (Maass et al, 2007; Harmsen et al.,2000). DNA Libraries were ligated into the pScreen expression vector and transformed in Top10 *E. coli* competent cells (Invitrogen) for expression by heatshock at 42°C for 1 minute. Antibody clones binding PD-L1 were subsequently isolated by ELISA screening against Human recombinant PD1 and PD-L1 (Randox and R&D Systems).

In total 223 sdAb clones (142 against PD1 and 81 against PD-L1) were isolated and sequenced using the Big Dye terminator kit v3.1 (Thermo-Fisher). Of 223 clones sequenced, 77 clones (53 against PD1 and 24 against PD-L1) were subsequently selected on the basis of having substantially different DNA sequences to undergo scaled-up expression and characterisation assessment.

### Example 2 - Pilot expressions

77 clones selected on the basis of sequence diversity were expressed in scaled up 200ml cultures as follows. 1µl of 5µg/ml pScreen plasmid mini-prep containing PD1/PD-L1 sdAb sequence was transformed into Top10 *E. coli* competent cells (Invitrogen) for expression by heatshock at 42°C for 1 minute. Cells were subsequently spread on agar plates containing 50µg/ml of ampicillin (Sigma) and incubated overnight at 37°C. The following morning colonies of each clone were inoculated into 200ml of TB media (Melford) and grown up at 37 degrees. The following day cultures were lysed using Bugbuster cell lysis reagent and purified by immobilised metal affinity chromatography (IMAC) using Talon resin (Takara). SdAb preparations were then quantified at 280nm and 2µg of each antibody was electrophoresed on a 12 % polyacrylamide gel via standard methods. Antibody purities were assigned based on visual inspection of the electrophoresed gels (See Table 1 for results of selected clones).

**Table 1 Antibody purities for selected clones**

| **Clone ID** | **Target** | **Yield (mg)** | **Purity (%)** |
|---|---|---|---|
| 32.1A1 | PD-L1 | 2.51 | 85% |
| 47.9E3 | PD1 | 2.70 | 40% |
| 32.1C7 | PD-L1 | 0.234 | 80% |
| 46.2D2 | PD1 | 1.82 | 60% |
| 46.2B12 | PD1 | 0.47 | 70% |
| 46.3C10 | PD1 | 0.714 | 70% |
| 46.4B10 | PD1 | 0.424 | 70% |

### Example 3 - PD1/PD-L1 Inhibition ELISA assay

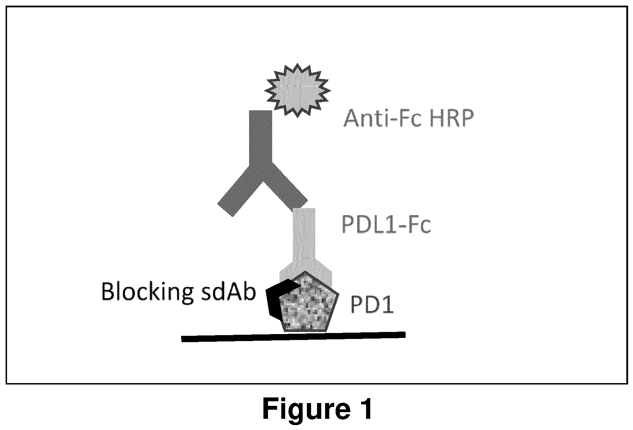

A PD1/PD-L1 inhibition assay was developed to test all 77 clones for inhibition of the PD1/PD-L1 interaction. In short, an ELISA plate was coated overnight at 4°C with 1 µg/mL of recombinant human PD1 (Randox) in PBS pH7.4 (Figure 1). The following day, the contents of the plate were discarded, and the plate was blocked with 300 µL/well of blocking reagent (Roche Cat# 11921681001) and incubated at 37°C for 1h. Post incubation, the blocking reagent was discarded and 50 µL of 60 ng/mL of PDL1-Fc (RnD Sys Cat# 156-B7) was added to each well and incubated for 1 hour at 37ºC. Following incubation, 50 µL per well of serially diluted (10,000 to 0.1 ng/mL) anti-PD1 or anti-PDL1 sdAb clones were added to test for blocking of the PD1/PD-L1 interaction. The plate was incubated at 37°C for 1h. This was followed by 6 x 300 µL/well wash with TBS-T followed by addition of 1/1000 dilution of goat anti-human IgG HRP conjugate (Abcam Ab97225) detector antibody. The plate was once more washed with 6 x 300 µL/well of TBS-T and the signal was then developed by adding 50 µL /well of TMB reagent and incubating at room temperature for 20 min in the dark. Signal development was stopped by addition of 50 µL of 2N sulphuric acid. The optical density of each well was read at OD450nm.

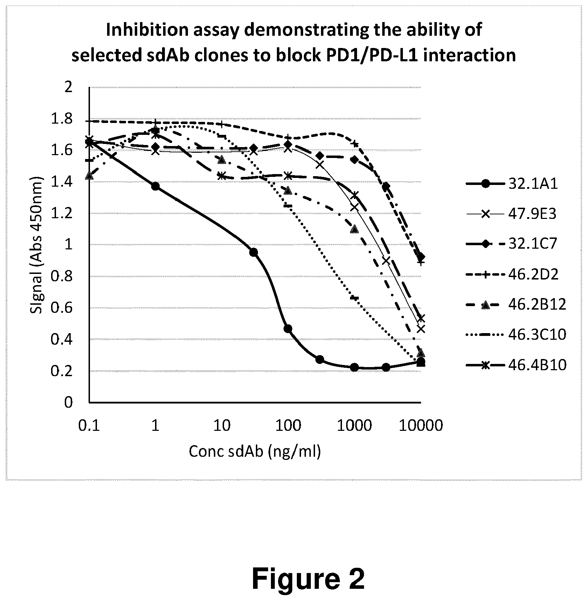

The results of this assay demonstrated that of the 77 clones tested for inhibition of PD1/PD-L1 binding only 7 clones (9.1%) demonstrated positive inhibition of PD1/PD-L1 binding. Of the seven blocking clones only one clone (clone 32.1A1) gave total inhibition of PD1/PD-L1 binding at a concentration of 1 µg/ml (See Figure 2 for blocking results, see Table 2 for sequence of all blocking clones).

**Table 2 - CDR sequences of selected clones**

| **Clone ID** | **Target** | **CDR1** | **CDR2** | **CDR3** |
|---|---|---|---|---|
| 32.1 A1 | PD-L1 | RTFREYGMG | TISSSGSYSY | AASSLLRGSSSRAESYDS |
| 47.9E3 | PD1 | SIFRGNAMV | TITNGGSTN | NDVIRGESGIYYPLNY |
| 32.1 C7 | PD-L1 | LAFATHTMG | GAPWNDNIE | AVTTRLLRTSYAEQYPY |
| 46.2D2 | PD1 | STFSNIYMG | RISSGGSTN | MRLISGASGWFPLDY |
| 46.2B12 | PD1 | RTVSNDDVG | RIDSDGSTN | MRLVSGASGWFPLDY |
| 46.3C10 | PD1 | TTFSNNDMG | RISSGGSTT | MRLVLGTSGWFPLDY |
| 46.4B10 | PD1 | ISNTENTMG | LISNSGLITH | YISTDPPT |
| 46.4H7 | PD1 | STFSNDYIG | RISSSGSTN | MREISGASGWFPLDY |
| 46.2D2.D12.D5 | PD1 | ISNTENTMG | LISNSGLITH | YISTEPPT |
| 46.2D2.H7 | PD1 | STFSNPYMG | RISSGGSTN | MRLISGASGWFPLDY |
| 32.2F7 | PD-L1 | RTFREYGMG | TISSSGSYTY | AASSLLRGSSSRAEPYDS |

### Example 4 - Further clone-assessment

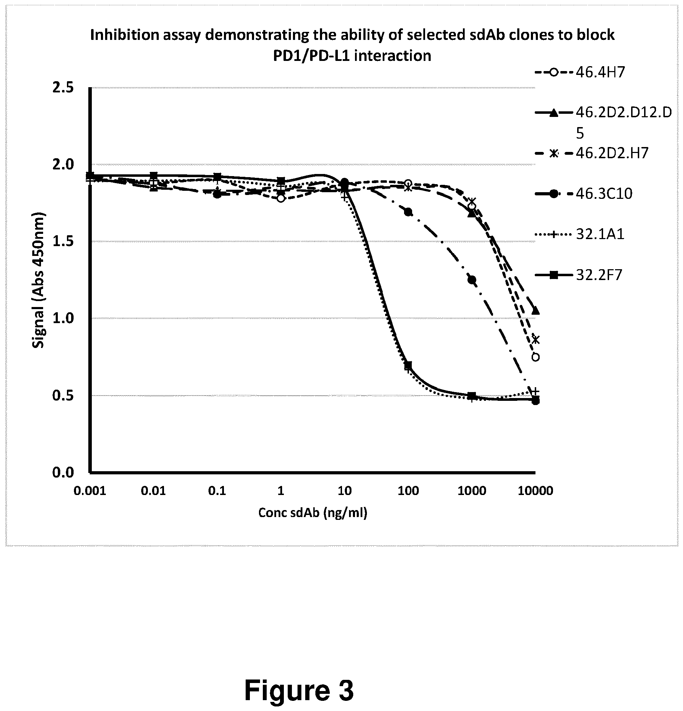

Following the described blocking-assay, the sequence database containing the alignment of all 223 sequences was scrutinised to identify clones that shared similar sequences to those clones identified in the above assay, which successfully blocked the PD1/PDL1 interaction. In all, four further clone sequences were identified which were largely similar to those blocking clones already identified including one clone 32.2F7 which differed from the lead blocking clone 32.1A1 by only four residues (including one residue substitution in CDR2 and one residue substitution in CDR3). These four clones (46.4H7, 46.2D2.D12.D5, 46.2D2.H7 and 32.2F7) were subsequently expressed and purified as described previously before being assessed in the same blocking assay described above using the lead PD1 and PD-L1 blocking antibodies 32.1A1 and 46.3C10 as positive controls. The results demonstrated that the lead anti-PDL1 clone 32.1A1 gave a blocking effect which was similar as compared to its close homologue 32.2F7, whilst none of the new anti-PD1 clones demonstrated blocking equivalent or better than the lead PD1 binding antibody 46.3C10 (Figure 3).

### Example 5 - Measurement of Binding affinity of clone 32.1A1

On an Octet Red96 instrument (FortéBio), recombinant Human PDL1-Fc chimeric protein (R&D Systems) was bound to the surface of Protein G biosensors (FortéBio) for up to 5 minutes, until a minimum binding response of 1.0 nM was achieved. Sensors were dipped into assay buffer for one minute before exposure to the anti-PDL1 single domain antibody 32.1A1 at a range of concentrations from 500 ng/mL to 7.81 ng/mL. After 5 minutes of incubation in the presence of sdAb, sensors were incubated in assay buffer for 15 minutes. The data was acquired in a PBS-based assay buffer (pH 7.40) at 30°C, in 96 well plates constantly agitated at 1000 rpm. Data traces were analysed using Octet Data Analysis software (version 10) by subtracting a reference trace (PD-L1 loaded sensor without sdAb analyte) and applying a Global Fit which assumed a 1:1 binding model giving a binding affinity (Kd) of 0.47nM. This figure compares favourably with that of 0.7nM stated for the commercial anti-PDL1 product Avelumab as stated in the FDA Center for Drug Evaluation and Research review (Application Number 761049Orig1s000).

### Example 6 - Humanisation and testing of clone 32.1A1

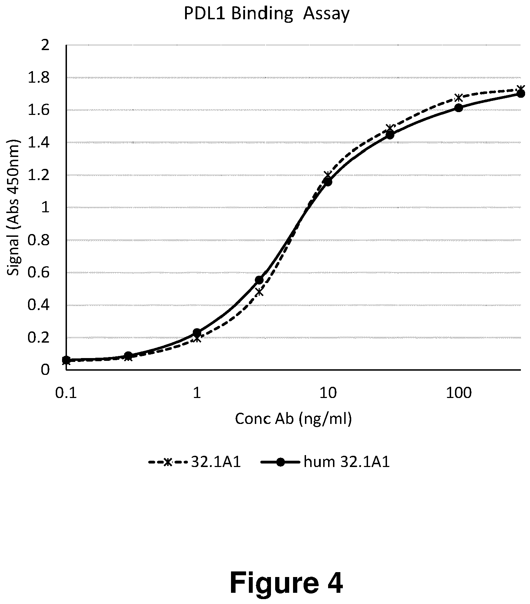

The CDRs of the lead blocking clone 32.1A1 were grafted into a model Human VH3 framework sequence and the humanised sequence was synthesized and cloned into the expression vector pScreen. Following expression of the clone using the methods outlined above the humanised clone was compared to the original clone 32.1A1 in a binding ELISA whereby PD-L1 was coated on the surface of an ELISA plate, blocked and probed with a gradient of each of the humanised and non-humanised antibody fragments. Finally, the ELISA plate was again washed and detected with a secondary detector antibody, anti-Myc HRP (abcam). The results demonstrated that the two antibodies were functionally equivalent and that full binding was retained following humanisation (Figure 4).

**Table 3 - Comparison signal output from binding ELISAs for clone 32.1A1 and its humanised derivative hum-32.1A1.**

| **Conc. sdAb (ng/ml)** | **32.1 A1 Mean Signal** | **hum-32.1A1 Mean Signal** |
|---|---|---|
| 0.1 | 0.055 | 0.0605 |
| 0.3 | 0.079 | 0.088 |
| 1 | 0.195 | 0.231 |
| 3 | 0.48 | 0.553 |
| 10 | 1.199 | 1.1575 |
| 30 | 1.486 | 1.4455 |
| 100 | 1.676 | 1.6135 |
| 300 | 1.728 | 1.7015 |

### Example 7 - PD1/PD-L1 Inhibition ELISA assay to benchmark against Avelumab

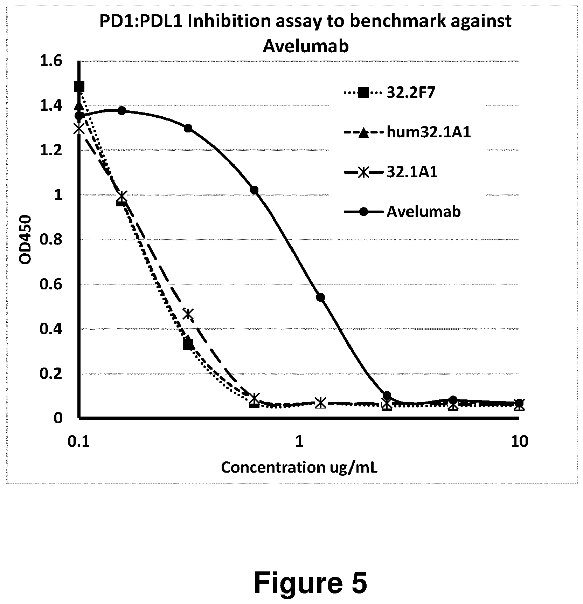

In order to benchmark the lead clone 32.1A1 and its derivatives against the commercially available therapeutic antibody Avelumab developed by Merck, it was necessary to use an assay format, which didn't employ an anti-IgG detector antibody. To this end, a commercial PD1 :PDL1 inhibition assay was sourced from Acro Biosystems (Cat# EP-101). In brief, this assay has PDL1 coated on the surface whilst biotinylated PD1 ligand is detected using a streptavidin-HRP conjugate. We used the assay to compare the blocking of 32.1A1, its humanised derivative hum32.1A1 and its close relative 32.2F7 to Avelumab (Creative Biolabs, Cat# TAB004ML). The results demonstrated that all three of the single domain antibodies tested gave more than ten times the amount of blocking as compared to Avelumab at similar concentrations (Figure 5).

### Example 8 - Cell based Inhibition assay

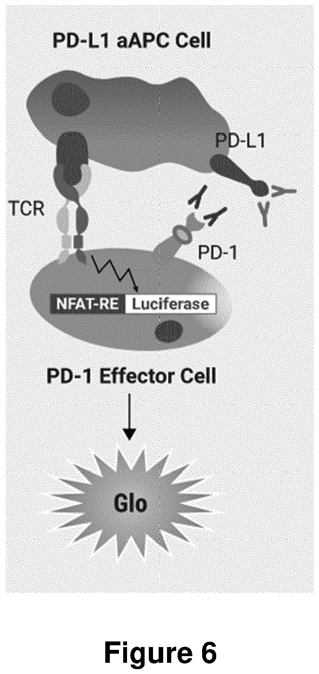

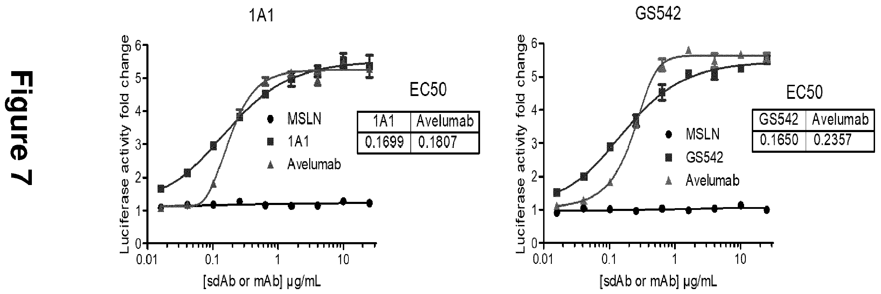

The two lead PD1 and PD-L1 blocking clones as identified by the ELISA Inhibition assay were tested using the Promega PD1/PD-L1 Blockade Bioassay (Cat# J1250), a commercial cellular assay to test the inhibition of PD1/PD-L1 interaction on the cell surface (Figure 6). The results of this assay also demonstrated the anti-PDL1 clone 32.1A1 to have superior blocking in this cell-based assay format than the commercial therapeutic antibody Avelumab (Figure 7).

### Example 9 - Stressed Stability and Solubility Studies

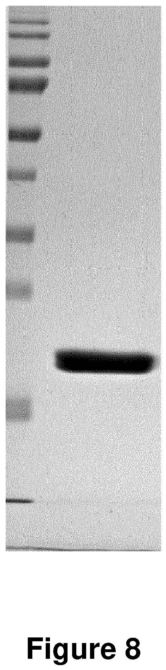

A larger scale 6 Litre expression was performed of the humanised 32.1A1 clone with a view to performing a higher stringency purification of the anti-PDL1 32.1A1 sdAb. In short, expressions were carried out as previously described with the exception that the 200ml culture was inoculated into a further 6 litres of TB prior to overnight incubation. The next day, following extraction of the scFv and purification by IMAC on Talon resin, the eluate was subsequently polished by size exclusion chromatography on a Hiload 26/600 superdex 75 PG column (GE Healthcare). The fractions corresponding to the sdAb monomer were subsequently pooled and electrophoresed on an SDS-PAGE gel according to standard methods. (Figure 8)

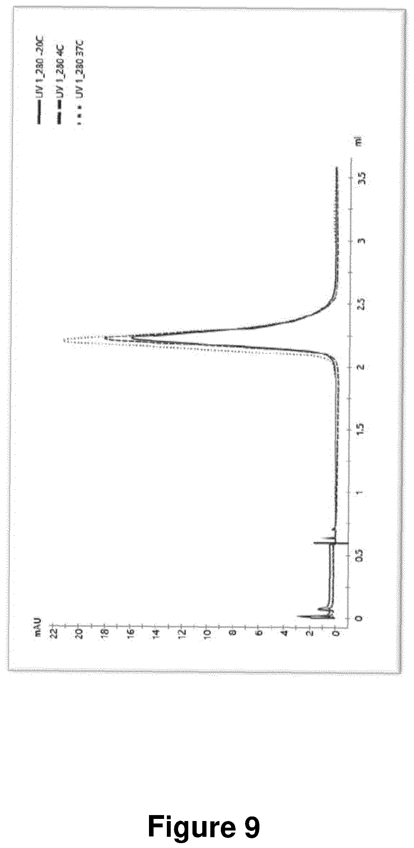

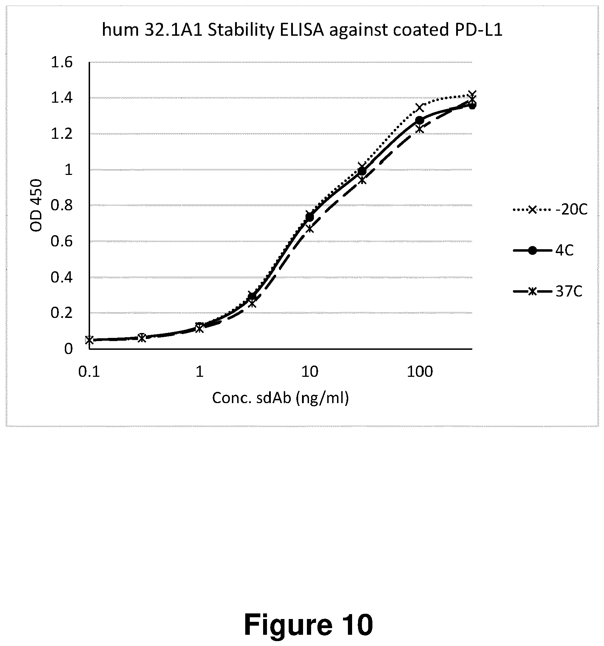

Subsequently three aliquots of the purified humanised 32.1A1 were stressed by storage for one week at -20, 4 and 37°C. Following storage at the different temperatures, 50µg of each aliquot was analysed on a superdex 75 Increase 5/150 GL size exclusion column to check for the formation of aggregates. The results of this assessment showed no evidence of aggregation at any of the storage temperatures tested. (Figure 9). Simultaneous to this assessment a binding assay was carried out to test the binding capability of the antibody following storage at each of the three temperatures. In short PD-L1 was coated on the surface of an ELISA plate, blocked and probed with a gradient of the antibody which had been stored at each of the three temperatures. Finally, the ELISA plate was again washed and detected with anti-Myc HRP (abcam) secondary detector antibody. The results of this assay demonstrated that no significant loss of binding had occurred following storage at any of the temperatures tested (Figure 10).

### Example 10 - Cross-reactivity Studies

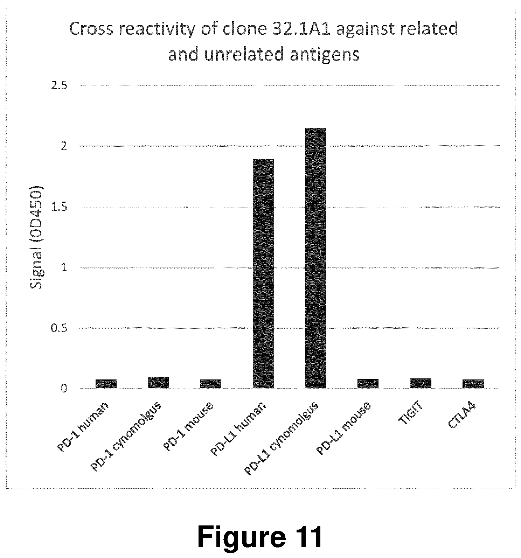

Human PD1, PD-L1, CTLA4 and TIGIT (all Randox), cynomolgus and mouse PD-L1 (both Sino Biological) and cynomolgus and mouse PD1 (both R&D Systems) were coated and blocked on the surface of an ELISA plate. The plate was probed with a 30ng/ml dilution of 32.1A1 antibody and incubated with shaking at 37ºC for 1 hour. Finally, the ELISA plate was washed and detected with anti-Myc HRP (abcam) secondary detector antibody. The results of this assay demonstrated binding of the 32.1A1 clone to human and cynomolgus PD-L1 but not to mouse PD-L1 or to any of the other unrelated antigens (See Figure 11).

### Sequences forming part of the description:

**SEQ ID NO: 1 (CDR1 of Clone ID 31.1A1)**
   RTFREYGMG
**SEQ ID NO: 2 (CDR2 of Clone ID 31.1A1)**
   TISSSGSYSY
**SEQ ID NO: 3 (CDR3 of Clone ID 31.1A1)**
   AASSLLRGSSSRAESYDS
**SEQ ID NO: 4 (CDR1 of Clone ID 32.2F7)**
   RTFREYGMG
**SEQ ID NO: 5 (CDR2 of Clone ID 32.2F7)**
   TISSSGSYTY
**SEQ ID NO: 6 (CDR3 of Clone ID 32.2F7)**
   AASSLLRGSSSRAEPYDS

SEQ ID NO: 7 (full-length antibody-CDRs 1-3)

QVQLVESGGGLVQPGGSLRLSCAASGRTFREYGMGWFRQAPGKGLEWVATI
SSSGSYSYYADSVKGRFTISRDNSKNTLYLQMNSLRAEDTAVYYCAASSLLRG
SSSRAESYDSWGQGTLVTVSS

SEQ ID NO: 8 (full-length antibody-CDRs 4-6)

QVQLVESGGGLVQPGGSLRLSCAASGRTFREYGMGWFRQAPGKGLEWVATI
SSSGSYTYYADSVKGRFTISRDNSKNTLYLQMNSLRAEDTAVYYCAASSLLRG
SSSRAEPYDSWGQGTLVTVSS

### References

1. Okazaki T, Honjo T. PD-1 and PD-1 ligands: from discovery to clinical application. Int Immunol. 2007;19(7):813-824.
2. Maass DR, Sepulveda J, Pernthaner A, and Shoemaker CB 2007 Alpaca, Lama pacos as a convenient source of recombinant camelid heavy chains. J. Immunol. Methods 2007: 324 (1-2):13-25.
3. Harmsen MM, Ruuls RC, Nijman IJ, Niewold TA, Frenken LG, de Geus B. 2000: Llama heavy chain V regions consist of at least four distinct subfamilies revealing novel sequence features. Mol. Immunol. 37 (2000): 579-590.

## Claims

1. An antibody or antigen-binding portion thereof, wherein the antibody or antigen-binding portion thereof has at least 80 % sequence identity to SEQ ID NO: 7 and wherein the antibody or antigen-binding portion thereof can specifically bind a programmed death-ligand 1 (PD-L1) polypeptide.

2. The antibody or antigen-binding portion thereof of claim 1, wherein the antibody or antigen-binding portion thereof comprises a heavy chain CDR having at least 70 % sequence identity to SEQ ID NO: 3.

3. The antibody or antigen-binding portion thereof of claim 1 or 2, wherein the antibody or antigen-binding portion thereof further comprises a heavy chain CDR having at least 70 % sequence identity to SEQ ID NO: 1 and/or comprises a heavy chain CDR having at least 70 % sequence identity to SEQ ID NO: 2.

4. An antibody or antigen-binding portion thereof, wherein the antibody or antigen-binding portion thereof comprises one or more heavy chain CDRs, wherein (i) the heavy chain CDR1 has at least 70% sequence identity to SED ID NO: 1, (ii) the heavy chain CDR2 has at least 70% sequence identity to SEQ ID NO: 2 and (iii) the heavy chain CDR3 has at least 70% sequence identity to SEQ ID NO:3, wherein the antibody or antigen-binding portion thereof can specifically bind a programmed death-ligand 1 polypeptide.

5. An antibody or antigen-binding portion thereof, wherein the antibody or antigen-binding portion thereof comprises a heavy chain CDR having at least 70 % sequence identity to SEQ ID NO: 3.

6. The antibody or antigen-binding portion thereof of claims 4 or 5, wherein the antibody or antigen-binding portion thereof has at least 80 % sequence identity to SEQ ID NO: 7, preferably at least 85 % sequence identity to SEQ ID NO: 7, more preferably at least 90 % sequence identity to SEQ ID NO: 7, even more preferably at least 95 % sequence identity to SEQ ID NO: 7.

7. The antibody or antigen-binding portion thereof according to any one of claims 1 to 6, wherein the antibody or antigen-binding portion thereof has a Kd value of between 0.3 and 0.6 nM, preferably between 0.4 and 0.6 nM, even more preferably between 0.4 and 0.5 nM.

8. The antibody or antigen-binding portion thereof according to any one of claims 1 to 7, wherein the antibody or antigen-binding portion thereof is a single domain antibody, preferably wherein the antibody or antigen-binding portion thereof is a single heavy-chain variable domain antibody.

9. The antibody or antigen-binding portion thereof according to any one of claims 1 to 8, wherein the antibody or antigen-binding portion thereof is a human or humanised antibody or antigen-binding portion thereof.

10. The antibody or antigen-binding portion thereof according to any one of claims 1 to 9, wherein the antibody or antigen-binding portion thereof forms part of a fusion protein or a bispecific antibody.

11. A nucleic acid encoding the antibody or antigen-binding portion thereof of claims 1 to 10, or a complementary sequence thereof.

12. The antibody or antigen-binding portion or nucleic acid thereof according to claims 1 to 11 for use in therapy.

13. The antibody or antigen-binding portion or nucleic acid thereof according to claims 1 to 11 for use in the treatment of cancer, preferably wherein the cancer is a tumour present within the central nervous system (CNS) or wherein the cancer is a solid tumour.

14. The antibody or antigen-binding portion or nucleic acid thereof according to claims 1 to 11 for use in the treatment of infectious disease.

15. A pharmaceutical composition comprising the antibody or antigen-binding portion thereof or the nucleic acid according to any one of claims 1-11, wherein the composition additionally includes a pharmaceutically acceptable carrier and/or a known therapeutic agent.
